(19) Europäisches Patentamt
European Patent Office
Office européen des brevets

(11) Veröffentlichungsnummer: **0 097 882 A2**

# (12) EUROPÄISCHE PATENTANMELDUNG

(21) Anmeldenummer: **83105896.1**

(22) Anmeldetag: **16.06.83**

(51) Int. Cl.³: **C 07 C 49/185**
C 07 C 49/258, C 07 C 49/86
C 07 C 49/355, C 07 C 49/175
C 07 C 45/56, C 07 D 317/26
C 07 D 319/06, C 07 D 307/12
//C07D405/06

(30) Priorität: **29.06.82 DE 3224130**

(43) Veröffentlichungstag der Anmeldung:
**11.01.84 Patentblatt 84/2**

(84) Benannte Vertragsstaaten:
**BE CH DE FR GB LI**

(71) Anmelder: **BAYER AG**
**Zentralbereich Patente, Marken und Lizenzen**
**D-5090 Leverkusen 1, Bayerwerk(DE)**

(72) Erfinder: **Ditgens, Klaus, Dr.**
**Claudiusweg 7**
**D-5600 Wuppertal 1(DE)**

(72) Erfinder: **Krämer, Wolfgang, Dr.**
**Am Eckbusch 39/45**
**D-5600 Wuppertal 1(DE)**

(72) Erfinder: **Elbe, Hans-Ludwig, Dr.**
**Dasnöckel 59**
**D-5600 Wuppertal 11(DE)**

## (54) Keto-aldehyd-Derivate und Verfahren zu ihrer Herstellung.

(57) Die Erfindung betrifft neue Keto-aldehyd-Derivate, ein Verfahren zu ihrer Herstellung und ihre Verwendung als Zwischenprodukte zur Synthese von Azolyl-ketonen und -alkoholen, welche fungizide und pflanzenwachstumsregulierende Eigenschaften besitzen.

Die Verbindungen der Formel

$$R^1 - \underset{\displaystyle CH_3}{\overset{\displaystyle CH_3}{\underset{|}{\overset{|}{C}}}} - CO - R^2 \qquad (I)$$

in welcher

$R^1$ und $R^2$ die in der Beschreibung angegebene Bedeutung besitzen,

können erhalten werden, wenn man Säurechloride mit 1-(N-Morpholino)-isobuten in Gegenwart eines Verdünnungsmittels umsetzt und hydrolysiert, sowie gegebenenfalls die so erhaltenen Ketoaldehyde in üblicher Weise an der Aldehydgruppe derivatisiert.

Die Verbindungen können z.B. durch Umsetzung mit Azolen, wie 1,2,4-Triazol, und anschließender Alkylierung in Azolyl-ketone und -alkohole mit fungizider und das Pflanzenwachstum regulierender Wirkung überführt werden.

EP 0 097 882 A2

Croydon Printing Company Ltd

BAYER AKTIENGESELLSCHAFT 5090 Leverkusen, Bayerwerk

Zentralbereich
Patente, Marken und Lizenzen Slr/AB
Ia/ZP

## Keto-aldehyd-Derivate und Verfahren zu ihrer Herstellung

Die vorliegende Erfindung betrifft neue Keto-aldehyd-Derivate, ein Verfahren zu ihrer Herstellung und ihre Verwendung als Zwischenprodukte zur Synthese von Azolylketonen und -alkoholen, welche fungizide und pflanzenwachstumsregulierende Eigenschaften besitzen.

Es ist bekannt geworden, daß bestimmte Azolyl-pentanone, wie beispielsweise 1-(4-Chlorphenyl)-4,4-dimethyl-2-(1,2,4-triazol-1-yl)-3-pentanon, gute fungizide Eigenschaften besitzen (vergleiche DE-OS 27 34 426). Außerdem ist bekannt geworden, daß bestimmte Azolylbutanone und -ole, wie beispielsweise alkylierte 3,3-Dimethyl-4-fluor(chlor)-1-(1,2,4-triazol-1-yl)-2-butanone und -ole, gute fungizide und pflanzenwachstumsregulierende Eigenschaften aufweisen (vergleiche DE-OS 29 51 164 [LeA 20 067] und DE-OS 29 51 163 [LeA 20 068]).
Die Wirkung all dieser Verbindungen ist jedoch in bestimmten Indikationsbereichen, insbesondere bei niedrigen Aufwandmengen und -konzentrationen, nicht immer ganz befriedigend.

Es wurden neue Keto-aldehyd-Derivate der allgemeinen Formel

$$R^1 - \overset{\displaystyle CH_3}{\underset{\displaystyle CH_3}{\overset{|}{\underset{|}{C}}}} - CO - R^2 \qquad (I)$$

in welcher

$R^1$ für die -CH=O-Gruppe und deren Derivate steht und

$R^2$ für Halogenalkyl, gegebenenfalls substituiertes Phenylalkyl, gegebenenfalls substituiertes Phenylalkenyl, gegebenenfalls substituiertes Phenoxyalkyl, gegebenenfalls substituiertes Phenylthioalkyl, substituiertes Phenoxy; für durch Halogen, Alkyl, Halogenalkyl, Halogenalkoxy, Halogenalkylthio, Phenyl oder Phenoxy substituiertes Phenyl, gegebenenfalls substituiertes Cycloalkyl, gegebenenfalls substituiertes Cycloalkylalkyl, Alkoxycarbonyl oder für einen Heterocyclus steht,

gefunden.

Weiterhin wurde gefunden, daß man die neuen Keto-aldehyd-Derivate der allgemeinen Formel (I) erhält, indem man Säurechloride der Formel

$$Cl - CO - R^2 \qquad (II)$$

in welcher

$R^2$ die oben angegebene Bedeutung hat,

mit 1-(N-Morpholino)-isobuten der Formel

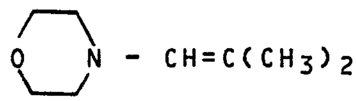 (III)

in Gegenwart eines Verdünnungsmittels umsetzt und hydrolisiert, sowie gegebenenfalls die so erhaltenen Keto-aldehyde der Formel

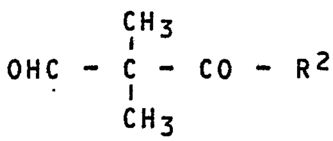 (Ia)

in welcher

$R^2$ die oben angegebene Bedeutung hat,

in üblicher Weise an der Aldehydgruppe derivatisiert.

Die neuen Keto-Derivate sind interessante Zwischenprodukte zur Herstellung von Pflanzenschutz-Wirkstoffen. So eignen sich die Stoffe der Formel (I) als Ausgangsprodukte zur Synthese von Azolyl-ketonen und -alkoholen, welche sehr gute fungizide und pflanzenwachstumsregulierende Wirksamkeit besitzen.

Ueberraschenderweise sind Azolyl-ketone und -alkohole, die sich aus erfindungsgemäßen Keto-aldehyd-Derivaten der Formel (I) z.B. durch Umsetzung mit Azolen und anschliessender Alkylierung, oder durch Halogenierung und Umsetzung mit Azolen sowie gegebenenfalls weiter anschließender Reduktion herstellen lassen, den oben genannten aus dem Stand der Technik bekannten Triazolyl-alkanonen und -olen bezüglich ihrer fungiziden und pflanzenwachstumsregulierenden Wirksamkeit überlegen.

Die erfindungsgemäßen Stoffe sind durch die Formel (I) allgemein definiert. In dieser Formel stehen vorzugsweise

$R^1$ für die -CH=O-Gruppe und deren Derivate, wie Oxime, Oximether und Acetale, wie beispielsweise Alkoxyiminomethyl mit 1 bis 4 Kohlenstoffatomen im Alkylteil, Dialkoxymethyl mit 1 bis 4 Kohlenstoffatomen in jedem Alkylteil, sowie gegebenenfalls substituierte Dioxolane und Dioxane; und

$R^2$ für Halogenalkyl mit 1 bis 4 Kohlenstoffatomen und 1 bis 5 gleichen oder verschiedenen Halogenatomen, wie vorzugsweise Fluor-, Brom- und Chloratomen; für gegebenenfalls einfach bis dreifach, gleich oder verschieden substituiertes Phenylalkyl mit 1 bis 2 Kohlenstoffatomen im Alkylteil, für gegebenenfalls einfach bis dreifach, gleich oder verschieden substituiertes Phenylalkenyl mit 2 bis 4 Kohlenstoffatomen im Alkenylteil, für gegebenenfalls einfach bis dreifach, gleich oder verschieden substituiertes Phenoxyalkyl oder Phenylthioalkyl mit jeweils 1 bis 2 Kohlenstoffatomen im Alkylteil und für einfach bis dreifach, gleich oder verschieden substituiertes Phenoxy, wobei jeweils als Phenylsubstituenten genannt seien: Halogen, Alkyl mit 1 bis 4 Kohlenstoffatomen, Alkoxy und Alkylthio mit jeweils 1 bis 2 Kohlenstoffatomen, Halogenalkyl, Halogenalkoxy und Halogenalkylthio mit jeweils 1 bis 2 Kohlenstoffatomen und 1 bis 5 gleichen oder verschiedenen Halogenatomen, wie vorzugsweise Fluor und Chloratomen, und schließlich Phenyl und Phenoxy; ferner für einfach bis dreifach, gleich oder verschieden substituiertes Phenyl, wobei als Substituenten genannt seien: Halogen, Alkyl mit 1 bis 4 Kohlenstoffatomen, Halogenalkyl, Halogenalkoxy und Halogenalkylthio mit jeweils 1 bis 2 Kohlenstoffatomen und 1 bis 5 gleichen oder verschiedenen Halogenatomen, wie vorzugsweise Fluor- und Chloratomen; weiterhin für

jeweils gegebenenfalls einfach bis dreifach, gleich oder verschieden durch Alkyl mit 1 bis 4 Kohlenstoffatomen substituiertes Cycloalkyl und Cycloalkylalkyl mit jeweils 3 bis 7 Kohlenstoffatomen im Cycloalkylteil und 1 bis 2 Kohlenstoffatomen im Alkylteil; sowie für Alkoxycarbonyl mit 1 bis 4 Kohlenstoffatomen im Alkylteil undfür einen 5- bis 6-gliedrigen Heterocyclus mit 1 bis 3 Heteroatomen, wie Sauerstoff, Schwefel oder Stickstoff.

Besonders bevorzugt sind diejenigen Verbindungen der Formel (I), in denen

$R^1$ für die -CH=O-Gruppe, Methoxyiminomethyl, Dimethoxymethyl, den gegebenenfalls durch Alkyl mit 1 bis 4 Kohlenstoffatomen oder Halogenphenoxymethyl substituierten Dioxolan- und 1,3-Dioxan-Rest steht; und

$R^2$ für Chlormethyl, Brommethyl, Fluormethyl, Dichlormethyl, Chlorethyl, Bromethyl und Chlorpropyl steht; für jeweils gegebenenfalls einfach bis zweifach, gleich oder verschieden substituiertes Benzyl, Phenylethyl, Phenylethenyl, Phenoxymethyl, Phenylthiomethyl und Phenoxy steht, wobei als jeweilige Phenylsubstituenten genannt seien: Fluor, Chlor, Methyl, Methoxy, Trifluormethyl, Phenyl und Phenoxy; ferner für einfach oder zweifach, gleich oder verschieden substituiertes Phenyl steht, wobei als Substituenten die oben genannten Phenylsubstituenten genannt seien; weiterhin für jeweils gegebenenfalls einfach bis zweifach, gleich oder verschieden durch Methyl oder Ethyl substituiertes Cyclopentyl, Cyclohexyl, Cyclopentylmethyl oder Cyclohexylmethyl steht; endlich noch für Methoxycarbonyl, Ethoxycarbonyl, Furyl und Thiophenyl steht.

Verwendet man beispielsweise Chloracetylchlorid und 1-(N-Morpholino)-isobuten als Ausgangsstoffe, so kann der Reaktionsablauf bei dem erfindungsgemäßen Verfahren durch das folgende Formelschema wiedergegeben werden:

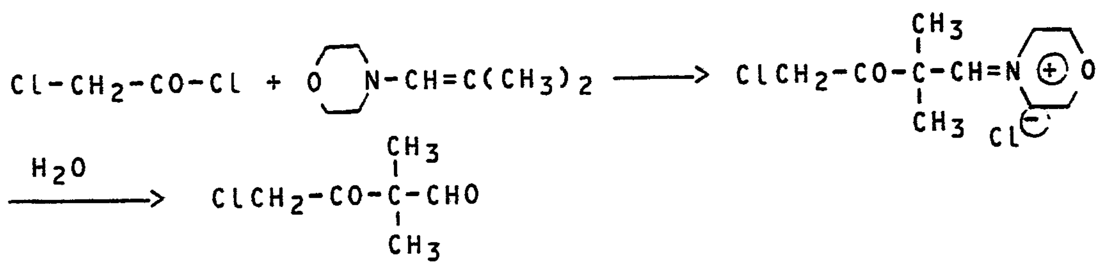


Die für die Durchführung des erfindungsgemäßen Verfahrens als Ausgangsstoffe benötigten Säurechloride sind durch die Formel (II) allgemein definiert. In dieser Formel steht $R^2$ vorzugsweise für diejenigen Bedeutungen, die bereits im Zusammenhang mit der Beschreibung der erfindungsgemäßen Stoffe der Formel (I) vorzugsweise für diesen Rest genannt wurden.

Die Säurechloride der Formel (II) sind allgemein bekannte Verbindungen der organischen Chemie.

Das außerdem für das erfindungsgemäße Verfahren als Ausgangsstoff zu verwendende 1-(N-Morpholino)-isobuten der Formel (III) und dessen Herstellung aus Morpholin und Isobutyraldehyd unter Wasserabspaltung ist bekannt (vgl. Angewandte Chemie 71, (1959) 521).

Für das erfindungsgemäße Verfahren kommen als Verdünnungsmittel inerte organische Lösungsmittel infrage. Hierzu gehören vorzugsweise Ether, wie Diethylether, Dioxan oder Tetrahydrofuran; Nitrile, wie Acetonitril; aromatische Kohlenwasserstoffe, wie Benzol und Toluol; sowie chlorierte Kohlenwasserstoffe, wie Methylenchlorid und Chloroform.

Die Reaktionstemperaturen können bei dem erfindungsgemäßen Verfahren in einem größeren Bereich variiert werden. Im allgemeinen arbeitet man bei Temperaturen zwischen 0 und 150°C, vorzugsweise zwischen + 10 und 120°C.

Bei der Durchführung des erfindungsgemäßen Verfahrens arbeitet man vorzugsweise in äquimolaren Mengen. Zur Isolierung der Endprodukte der Formel (I) wird das Reaktionsgemisch auf Wasser gegeben, die organische Phase abgetrennt und in üblicher Art und Weise aufgearbeitet.

Die erfindungsgemäßen Keto-aldehyde der Formel (Ia) können gegebenenfalls noch in bekannter Art und Weise an der Aldehydgruppe derivatisiert werden, wie z.B. durch Umsetzung mit Diolen in Gegenwart eines Verdünnungsmittels und in Gegenwart einer Säure als Katalysator.

Als Verdünnungsmittel kommen für die Derivatisierung mit Diolen inerte organische Lösungsmittel infrage. Hierzu gehören vorzugsweise aromatische Kohlenwasserstoffe, wie Benzol, Toluol oder Xylol; sowie halogenierte aliphatische und aromatische Kohlenwasserstoffe, wie Tetrachlorkohlenstoff, Chloroform, Methylenchlorid, Chlorbenzol oder Dichlorbenzol sowie Gemische dieser Lösungsmittel mit Alkoholen, wie z.B. Butanol. Es kann aber auch gegebenenfalls ein entsprechender Ueberschuß an Diol verwendet werden.

Die Derivatisierung mit Diolen wird in Gegenwart einer starken Säure als Katalysator durchgeführt. Hierzu ge-

hören vorzugsweise die Chlorwasserstoffsäure, Bromwasserstoffsäure, Schwefelsäure sowie insbesondere die p-Toluolsulfonsäure.

Die Reaktionstemperaturen können bei der Durchführung der Derivatisierung mit Diolen in einem größeren Bereich variiert werden. Im allgemeinen arbeitet man zwischen 40 und 180°C, vorzugsweise bei 80 bis 180°C. Gegebenenfalls kann auch unter erhöhtem Druck gearbeitet werden.

Bei der Durchführung der Derivatisierung mit Diolen setzt man auf 1 Mol der Verbindungen der Formel (Ia) vorzugsweise 1 bis 2 Mol Diol sowie katalytische Mengen Säure ein. Die Isolierung der entstehenden Verbindungen der Formel (I) erfolgt in üblicher Art und Weise.

Die Derivatisierung der Keto-aldehyde der Formel (Ia) an der Aldehydgruppe kann auch durch übliche Umsetzung mit (substituierten) Hydroxylaminen, vorzugsweise in Form ihrer Hydrochloride, gegebenenfalls in Gegenwart eines Säurebindemittels, wie beispielsweise Natriumacetat, und in Gegenwart eines Verdünnungsmittels, wie beispielsweise Alkohole oder Wasser bzw. Gemische beider, bei Temperaturen zwischen 50 und 100°C erfolgen.

Die erfindungsgemäßen Keto-aldehyd-Derivate der Formel (I) eignen sich als Zwischenprodukte zur Synthese von substituierten Azolyl-ketonen und -alkoholen, welche fungizide und pflanzenwuchsregulierende Wirksamkeit besitzen.

Solche substituierten Azolyl-ketone und -alkohole der Formel

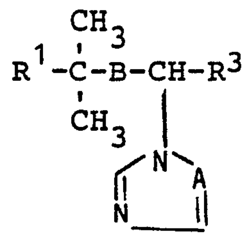 (IV)

in welcher

A für ein Stickstoffatom oder die CH-Gruppe steht,

B für die Keto- oder CH(OH)-Gruppe steht,

$R^1$ die oben angegebene Bedeutung hat und

$R^3$ für Alkyl, Alkenyl, Alkinyl, gegebenenfalls substituiertes Phenylalkyl, gegebenenfalls substituiertes Cycloalkyl oder gegebenenfalls substituiertes Cycloalkylalkyl steht,

lassen sich herstellen, indem man Keto-aldehyd-Derivate der Formel

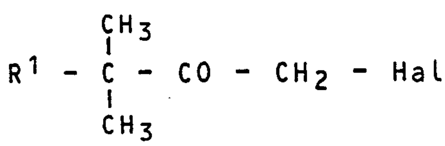 (Ib)

in welcher

$R^1$ die oben angegebene Bedeutung hat und

Hal für Chlor oder Brom steht,

zunächst in üblicher Weise mit 1,2,4-Triazol oder Imidazol in Gegenwart eines inerten organischen Lösungsmittels , wie beispielsweise Aceton, und in Gegenwart eines Säurebindemittels, wie beispielsweise Kaliumcarbonat, bei Temperaturen zwischen 20 und 150°C umsetzt; und die so erhaltenen Azolyl-Ketone der Formel

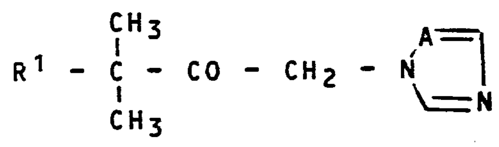 (V)

in welcher

A und $R^1$ die oben angegebene Bedeutung haben,

anschließend in üblicher Weise mit einem Alkylierungsmittel der Formel

$R^3 - Z$ (VI)

in welcher

$R^3$ die oben angegebene Bedeutung hat und

Z für eine elektronenanziehende Abgangsgruppierung, wie Halogen, p-Methylphenylsulfonyloxy oder Sulfat steht,

in Gegenwart eines inerten organischen Lösungsmittels, wie beispielsweise Dimethylformamid oder Dimethylsulfoxid, und in Gegenwart einer Base, wie beispielsweise

Kaliumhydroxid, oder in einem wäßrig-organischen Zweiphasensystem in Gegenwart eines Phasentransferkatalysators bei Temperaturen zwischen 20 und 100°C umsetzt; und gegebenenfalls die so erhaltenen substituierten Azolyl-Ketone der Formel

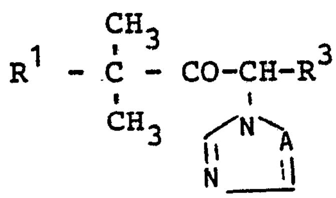

(IVa)

in welcher

A, $R^1$ und $R^3$ die oben angegebene Bedeutung haben,

in üblicher Weise reduziert, wie z.B. durch Umsetzung mit komplexen Hydriden, wie Natriumborhydrid oder Lithium-alanat, in Gegenwart eines polaren organischen Lösungsmittels, wie z.B. Alkohole, bei Temperaturen von ca. 0 bis 30°C; oder durch Umsetzung mit Aluminiumisopropylat in Gegenwart eines Verdünnungsmittels, wie z.B. Isopropanol,bei Temperaturen von ca. 20 bis 120°C.

An die so erhaltenen Verbindungen der Formel (IV) kann gegebenenfalls anschließend eine Säure addiert werden.

In manchen Fällen erweist es sich als vorteilhaft, die Verbindungen der Formel (IV) über ihre Salze in reiner Form zu erhalten.

Solche Azolyl-ketone der Formel

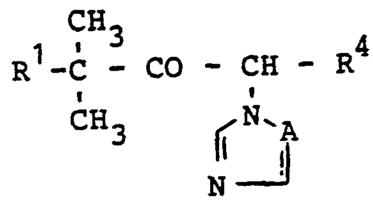 (IV b)

in welcher

A und $R^1$ die oben angegebene Bedeutung haben und

$R^4$ für gegebenenfalls substituiertes Phenylalkyl, gegebenenfalls substituiertes Cycloalkyl oder gegebenenfalls substituiertes Cycloalkylalkyl steht,

lassen sich auch herstellen, indem man Keto-aldehyd-Derivate der Formel

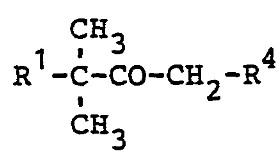

in welcher

$R^1$ und $R^4$ die oben angegebene Bedeutung haben,

zunächst in üblicher Weise halogeniert, wie z.B. durch Umsetzung mit Chlor, Brom oder Sulfurylchlorid in Gegenwart eines inerten organischen Lösungsmittels, wie z.B. Tetra-

chlorkohlenstoff, bei Temperaturen zwischen 0 und 40°C, und sodann die so erhaltenen Halogenketone der Formel

$$R^1-\overset{CH_3}{\underset{CH_3}{C}}-CO-\underset{Hal}{CH}-R^4 \qquad (VII)$$

in welcher

Hal, $R^1$ und $R^4$ die oben angegebene Bedeutung haben,

in üblicher Weise mit 1,2,4-Triazol oder Imidazol in Gegenwart eine inerten organischen Lösungsmittels, wie beispielsweise Aceton, und in Gegenwart eines Säurebindemittels, wie beispielsweise Kaliumcarbonat, bei Temperaturen zwischen 20 und 150°C umsetzt.

Die aus den erfindungsgemäßen Stoffen herstellbaren substituierten Azolyl-ketone und -alkohole der Formel (IV) besitzen gute fungizide und pflanzenwuchsregulierende Eigenschaften.

Herstellungsbeispiele

Beispiel 1

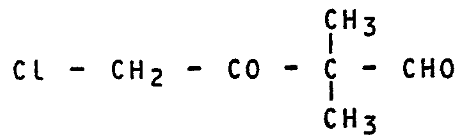

210g (1,5 Mol) 1-(N-Morpholino)-isobuten werden innerhalb einer Stunde zu 169,0g (1,5 Mol) Chloracetylchlorid, gelöst in 350 ml Diethylether, bei 5°C zugetropft. Nach beendeter Zugabe rührt man weitere 3 Stunden unter Rückflußkühlung. Die Lösung wird auf 100 g Eis gegossen, mit wäßriger Natriumhydrogencarbonatlösung auf pH 5 gebracht und die Etherphase abgetrennt. Die Wasserphase wird mit 100 ml Diethylether extrahiert, die organischen Phasen werden vereinigt, über Natriumsulfat getrocknet, das Lösungsmittel abdestilliert und der Rückstand im Wasserstrahlvakuum destilliert. Man erhält 136,4 g (61 % der Theorie) 4-Chlor-2,2-dimethyl-3-keto-butanal vom Siedepunkt 95-98°C/ 14 mbar.

Beispiel 2

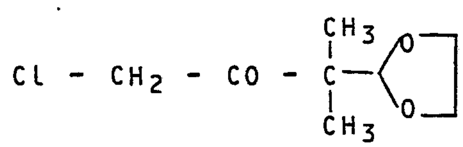

204g (1,38 Mol) 4-Chlor-2,2-dimethyl-3-keto-butanal (Beispiel 1) werden mit 93 g (1,5 Mol) Ethylenglykol und 0,7 g p-Toluolsulfonsäure in 400 ml Methylenchlorid während 3 Stunden am Wasserabscheider erhitzt. Die organische Phase wird mit 150 ml 5 %iger Natronlauge und danach mit 400 ml Wasser extrahiert. Das Lösungsmittel wird abdestilliert und der Rückstand im Wasserstrahlvakuum destilliert. Man erhält 211 g (79,8 % der Theorie) 1-Chlor-3-(dioxolan-2-yl)-3-methyl-butan-2-on vom Siedepunkt 127-128°C/ 14 mbar.

Beispiel 3

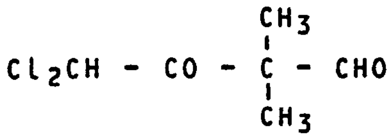

141g (1 Mol) 1-(N-Morpholino)-isobuten werden in 470 ml Diethylether gelöst. Unter Eiskühlung tropft man 147,5g (1 Mol) Dichloracetylchlorid langsam zu. Das Reaktionsgemisch wird anschließend 24 Stunden bei Raumtemperatur gerührt und danach mit 150 ml Wasser sowie Natriumhydrogencarbonatlösung versetzt, bis ein pH-Wert von 5 bis 6 erreicht ist. Man läßt eine weitere Stunde rühren und trennt danach die Etherphase ab. Die wässrige Phase wird mit Ether und anschließend mit Methylenchlorid extrahiert. Die vereinigten organischen Phasen werden über Natriumsulfat getrocknet, eingeengt und der Rückstand im Vakuum destilliert. Man erhält 155,6g (85 % der Theorie) 4-Dichlor-2,2-dimethyl-3-keto-butanal vom Siedepunkt 118°C/0,2 mbar.

Beispiel 4

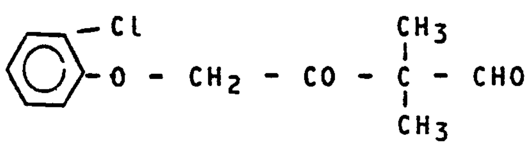

141g ( 1 Mol) 1-(N-Morpholino)-isobuten werden in 470 ml Diethylether gelöst. Unter Eiskühlung tropft man 205g (1 Mol) (2-Chlorphenoxy)-acetylchlorid langsam zu. Das Reaktionsgemisch wird anschließend 2 Stunden unter Rückfluß erhitzt. Man läßt abkühlen und saugt ab. Der Rückstand wird mit Wasser und Natriumhydrogencarbonatlösung versetzt, bis ein pH-Wert von 5 bis 6 erreicht ist. Die Reaktionslösung wird mit Ether und Methylenchlorid extrahiert. Die vereinigten organischen Phasen werden mit

Wasser gewaschen, über Natriumsulfat getrocknet und eingeengt. Man erhält 144,3g (60 % der Theorie) 4-(2-Chlorphenoxy)-2,2-dimethyl-3-keto-butanal vom Schmelzpunkt 50°C.

In analoger Weise und entsprechend dem erfindungsgemäßen Verfahren werden die in der folgenden Tabelle aufgeführten Verbindungen der Formel

$$R^1 - \underset{CH_3}{\overset{CH_3}{\underset{|}{\overset{|}{C}}}} - CO - R^2 \qquad (I)$$

erhalten:

| Bsp. Nr. | $R^1$ | $R^2$ | Physikal. Konstante |
|---|---|---|---|
| 5 | -CHO | 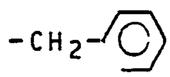 | Kp:90°C/ 0,02 mbar |
| 6 | -CHO | 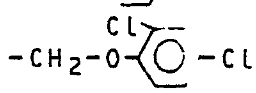 | Fp:50°C |
| 7 | 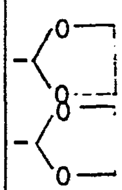 | 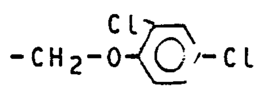 | Kp:171-73°C/ 0,1 mbar |
| 8 | | 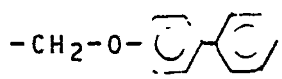 | Fp:58-60 |
| 9 | 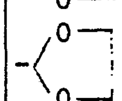 | 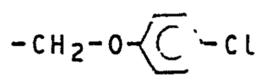 | Fp:57-59 |
| 10 | 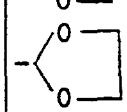 | 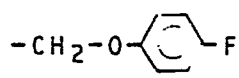 | Kp:143-45°C/ 0,1 mbar |
| 11 | 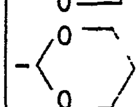 | 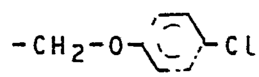 | Fp: 71-76°C |
| 12 | 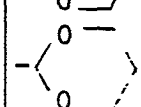 | 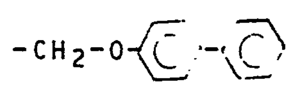 | Fp:82°C |
| 13 | -CHO | $-(CH_2)_3Cl$ | Kp:77°C/ 0,1 mbar |

| Bsp. Nr. | $R^1$ | $R^2$ | Physikal. Konstante |
|---|---|---|---|
| 14 | -CHO | $-CH_2CH_2Cl$ | Kp:58°C/ 11 mbar |
| 15 | -CHO | 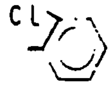 | Kp:77°C/ 0,15 mbar |
| 16 | -CHO | 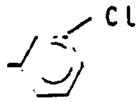 | Fp: 43°C |
| 17 | -CHO | 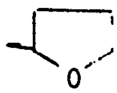 | Kp: 70°C/ 0,5 mbar |
| 18 | -CHO | 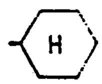 | Kp:85°C/ 0,3 mbar |
| 19 | -CHO | $-\overset{O}{\overset{\|}{C}}-OCH_3$ | Kp:110°C/ 11 mbar |
| 20 | -CHO | $-\overset{O}{\overset{\|}{C}}-OC_2H_5$ | Kp:91-94°C/ 10 mbar |
| 21 | -CHO | -O-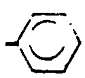 | Kp:76°C/ 0,01 mbar |
| 22 | -CHO | $-CH_2Br$ | Kp:70°C/ 1 mbar |
| 23 | -CHO | 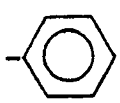$-CF_3$ | Kp: 124°C/ 11 mbar |
| 24 | 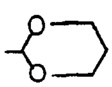 | $-CHCl_2$ | $n_D^{20}$: 1,4772 |
| 25 | 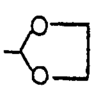 | $-CHCl_2$ | $n_D^{20}$: 1,4853 |

| Bsp. Nr. | $R^1$ | $R^3$ | Physikal. Konstante |
|---|---|---|---|
| 26 | $-CH{=}N-OCH_3$ | $-CH_2Cl$ | $n_D^{20}$: 1,4628 |
| 27 | $-CH{=}N-OCH_3$ | $-CHCl_2$ | $n_D^{20}$: 1,4737 |
| 28 | $-CH(OCH_3)_2$ | $-CH_2Cl$ | Kp: 117°C/12 mbar |
| 29 | 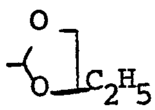 | $-CH_2Cl$ | Kp: 89°C/0,3 mbar |
| 30 | 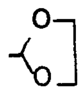 | 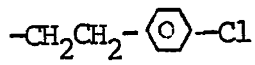 | Kp: 140-42°C/ 0,05 mbar |
| 31 | 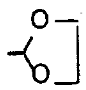 | 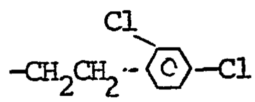 | Kp: 148°C/0,1 mbar |
| 32 | 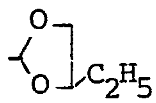 | 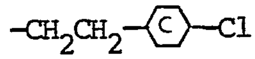 | Öl |
| 33 | 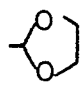 | 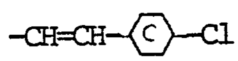 | Fp: 72-75°C |
| 34 | 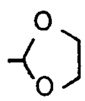 | 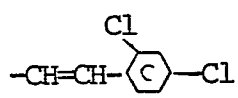 | Fp: 92-93°C |
| 35 | 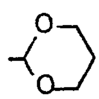 | 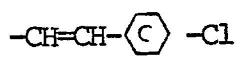 | Fp: 90°C |
| 36 | 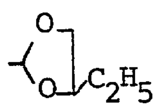 | 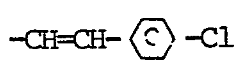 | Fp: 58°C |
| 37 | 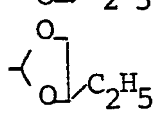 | 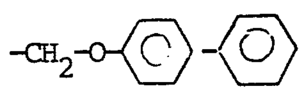 | Öl |
| 38 | 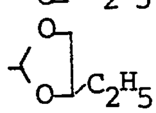 | 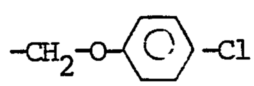 | Kp: 148°C/0,05 mbar |
| 39 | 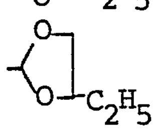 | 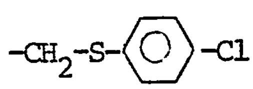 | Kp: 155-60°C/0,05 mbar |

| Bsp. Nr. | $R^1$ | $R^2$ | Physikal. Konst. |
|---|---|---|---|
| 40 | 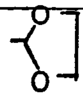 | 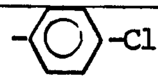 | Kp: 118-22 °C/0,05 mbar |
| 41 | 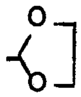 | 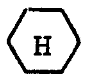 | Kp: 87 -90 °C/0,2mbar |
| 42 | 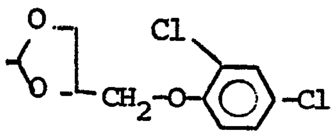 | $-CH_2Cl$ | Öl |

Herstellung des Folgeproduktes

Beispiel a

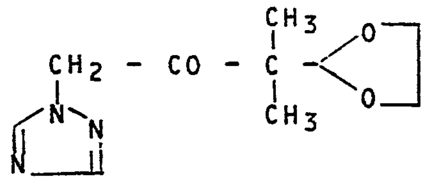

(V-1)

180,6g (0,94 Mol) 1-Chlor-3-(dioxolan-2-yl)-3-methyl-butan-2-on (Beispiel 2), 64,9g (0,94 Mol) 1,2,4-Triazol und 142,7g (1 Mol) Kaliumcarbonat werden in 1000 ml Methylethylketon während 16 Stunden unter Rückfluß erhitzt. Der Feststoff wird abgesaugt und das Lösungsmittel abdestilliert, der ölige Rückstand in 700 ml Methylenchlorid aufgenommen und die organische Phase zweimal mit je 1000 ml Wasser extrahiert. Von der organischen Phase wird das Lösungsmittel abdestilliert. Man erhält 151,3g (71,5 % der Theorie) 1-(1,2,4-Triazol-1-yl)-3-(dioxolan-2-yl)-3-methyl-butan-2-on als Oel mit gaschromatographischer Reinheit von 99 %.

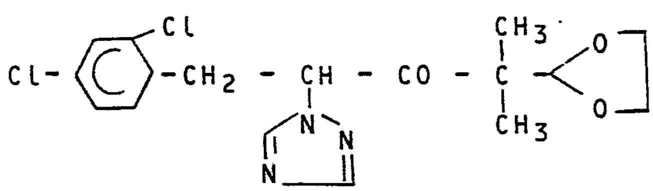

(IV-1)

x 1/2

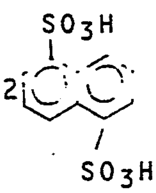

34g (0,15 Mol) 1-(1,2,4-Triazol-1-yl)-3-dioxolan-2-yl)-3-methyl-butan-2-on werden in 150 ml Dimethylsulfoxid vorgelegt, 8,4g gepulvertes Kaliumhydroxid bei 15°C zugegeben und 36 g (0,15 Mol) 2,3-Dichlorbenzylbromid in 30 ml Dimethylsulfoxid gelöst, zugetropft. Das Reaktionsgemisch wird bei 20°C 15 Stunden nachgerührt und die Suspension auf 500 ml Wasser gegossen. Man extrahiert mit 600 ml Methylenchlorid, wäscht die organische Phase zweimal mit je 1000 ml Wasser und destilliert das Lösungsmittel ab. Der ölige Rückstand wird in 600 ml Aceton gelöst

und mit 1,5-Naphthalindisulfonsäure in 60 ml Aceton versetzt. Bei 0°C erfolgt nach 6 Stunden Kristallisation. Man erhält 50g (38,6 % der Theorie) 1-(2,4-Dichlorphenyl)-2-(1,2,4-triazol-1-yl)-4-(dioxolan-2-yl)-4-methyl-pentan-3-on -1,5-naphthalindisulfonat vom Schmelzpunkt 199-201°C.

Beispiel b

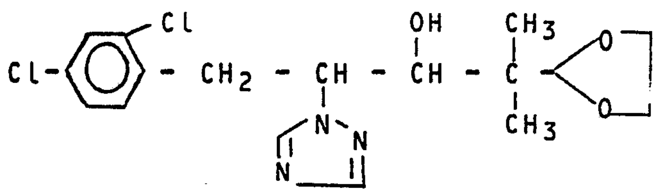

Beispiel (IV-2): B-Form*
Beispiel (IV-3): A-Form*

*A- und B-Form: die beiden möglichen geometrischen Isomeren

22,9g (0,06 Mol) 1-(2,4-Dichlorphenyl)-2-(1,2,4-triazol-1-yl)-4-(dioxolan-2-yl)-4-methyl-pentan-3-on werden in 100 ml Methanol gelöst, auf 0°C abgekühlt und portionsweise 2,95 g (0,078 Mol) Natriumborhydrid zugegeben. Man läßt 3 Stunden bei Raumtemperatur nachrühren, 15 ml konzentrierte Salzsäure zutropfen, weitere 3 Stunden nachrühren und gibt dann 500 ml gesättigte Natriumhydrogencarbonatlösung zu. Man extrahiert mit 400 ml Methylenchlorid, trennt die Phasen, wäscht die organische Phase zweimal mit je 1000 ml Wasser und destilliert das Lösungsmittel ab. Der Rückstand wird in 150 ml Diisopropylether aufgenommen und die Kristalle abgesaugt. Man erhält 1-(2,4-Dichlorphenyl)-2-(1,2,4-triazol-1-yl)-4-(dioxolan-2-yl)-4-methyl-pentan-3-ol als B-Form, vom Schmelzpunkt 178-181°C und nach Abdestillieren von ca. 100 ml Diisopropylether 8,9g als A-Form vom Schmelzpunkt 95-100°C.

Patentansprüche

1. Keto-aldehyd-Derivate der allgemeinen Formel

$$R^1 - \underset{\displaystyle CH_3}{\overset{\displaystyle CH_3}{\underset{|}{\overset{|}{C}}}} - CO - R^2 \qquad (I)$$

in welcher

$R^1$ für die -CH=O-Gruppe und deren Derivate steht und

$R^2$ für Halogenalkyl, gegebenenfalls substituiertes Phenylalkyl, gegebenenfalls substituiertes Phenylalkenyl, gegebenenfalls substituiertes Phenoxyalkyl, gegebenenfalls substituiertes Phenylthioalkyl, substituiertes Phenoxy, für durch Halogen, Alkyl, Halogenalkoxy, Halogenalkylthio, Phenyl oder Phenoxy substituiertes Phenyl, gegebenenfalls substituiertes Cycloalkyl, gegebenenfalls substituiertes Cycloalkylalkyl, Alkoxycarbonyl oder für einen Heterocyclus steht.

2. Verbindungen der allgemeinen Formel I in Anspruch 1, wobei

$R^1$ für die -CH=O-Gruppe, Methoxyiminomethyl, Dimethoxymethyl, den gegebenenfalls durch Alkyl mit 1 bis 4 Kohlenstoffatomen oder Halogenphenoxymethyl substituierten Dioxolan- und 1,3-Dioxan-Rest steht; und

$R^2$ für Chlormethyl, Brommethyl, Fluormethyl, Dichlormethyl, Chlorethyl, Bromethyl und Chlorpropyl steht; für jeweils gegebenenfalls einfach bis zweifach, gleich oder verschieden substituiertes Benzyl, Phenylethyl, Phenylethenyl, Phenoxymethyl, Phenylthiomethyl und Phenoxy steht, wobei als jeweilige Phenylsubstituenten genannt seien: Fluor, Chlor, Methyl, Methoxy, Trifluormethyl, Phenyl und Phenoxy; ferner für einfach oder zweifach gleich oder verschieden substituiertes Phenyl steht, wobei als Substituenten die oben genannten Phenylsubstituenten genannt seien; weiterhin für jeweils gegebenenfalls einfach bis zweifach, gleich oder verschieden durch Methyl oder Ethyl substituiertes Cyclopentyl, Cyclohexyl, Cyclopentylmethyl oder Cyclohexylmethyl steht, endlich noch für Methoxycarbonyl, Ethoxycarbonyl, Furyl und Thiophenyl steht.

3. Verfahren zur Herstellung von Keto-aldehyd-Derivaten der allgemeinen Formel

$$R^1 - \underset{\underset{CH_3}{|}}{\overset{\overset{CH_3}{|}}{C}} - CO - R^2 \qquad (I)$$

in welcher

$R^1$ für die -CH=O-Gruppe und deren Derivate steht und

$R^2$ für Halogenalkyl, gegebenenfalls substituiertes Phenylalkyl, gegebenenfalls substituiertes Phenylalkenyl, gegebenenfalls

substituiertes Phenoxyalkyl, gegebenenfalls substituiertes Phenylthioalkyl, substituiertes Phenoxy, für durch Halogen, Alkyl, Halogenalkyl, Halogenalkoxy, Halogenalkylthio, Phenyl oder Phenoxy substituiertes Phenyl, gegebenenfalls substituiertes Cycloalkyl, gegebenenfalls substituiertes Cycloalkylalkyl, Alkoxycarbonyl oder für einen Heterocyclus steht,

dadurch gekennzeichnet, daß man Säurechloride der Formel

$$Cl - CO - R^2 \qquad (II)$$

in welcher

$R^2$ die oben angegebene Bedeutung hat,

mit 1-(N-Morpholino)-isobuten der Formel

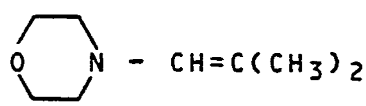

(III)

in Gegenwart eines Verdünnungsmittels umsetzt und hydrolisiert, sowie gegebenenfalls die so erhaltenen Ketoaldehyde der Formel

$$OHC - \underset{CH_3}{\underset{|}{\overset{CH_3}{\overset{|}{C}}}} - CO - R^2 \qquad (Ia)$$

in welcher
$R^2$ die oben angegebene Bedeutung hat,

in üblicher Weise an der Aldehydgruppe derivatisiert.

4. Verwendung von Keto-aldehyd-Derivaten der Formel I in Ansprüchen 1 und 3 als Zwischenprodukte zur Herstellung von substituierten Azolyl-ketonen und -alkoholen mit fungizider und das Pflanzenwachstum regulierender Wirkung.